# EUROPÄISCHE PATENTANMELDUNG

(11) **EP 2 878 313 A1**
(43) Veröffentlichungstag der Anmeldung: **03.06.2015**
(21) Anmeldenummer: 13195012.3
(22) Anmeldetag: 29.11.2013
(51) Int. Cl.: A61L 15/40, A61K 9/70, A61L 15/58

(54) **Heftpflaster enthaltend ätherische Öle und Verfahren zu seiner Herstellung**

(71) Anmelder: LTS LOHMANN Therapie-Systeme AG, 56626 Andernach (DE)
(72) Erfinder: Die Erfindernennung liegt noch nicht vor
(74) Vertreter: Schweitzer, Klaus

(57) **Zusammenfassung**

Die Erfindung betrifft ein Nasenpflaster zur Behandlung von Schnupfenbeschwerden, welches zumindest folgende Schichten aufweist
(a) mindestens eine haftklebende und hautfreundliche Polymerschicht,
(b) mindestens eine für ätherische Öle undurchlässige Zwischenschicht,
(c) mindestens eine weitere haftklebende Polymerschicht und
(d) mindestens eine ätherische Öle enthaltende Rückschicht.

## Beschreibung

Die vorliegende Erfindung betrifft ein Heftpflaster, bevorzugt in Form eines Nasenpflasters, welches einen speziellen mehrschichtigen Aufbau aufweist, ätherische Öle und bevorzugt auch Dexpanthenol enthält, welches insbesondere als hautfreundlicher Klebrigmacher einer haftklebenden Polymerschicht Verwendung findet. Ferner betrifft die Erfindung ein Verfahren zur Herstellung des Pflasters. Das erfindungsgemäße Heftpflaster, ein Verfahren zu seiner Herstellung und die erfindungsgemäße Verwendung von Dexpanthenol werden in den unabhängigen Ansprüchen beschrieben, bevorzugte Varianten sind in den jeweils abhängigen Ansprüchen ausgeführt.

Es ist im Stand der Technik bekannt, dass ätherische Öle durch ihre antiphlogistische Wirkung z. B. Schnupfenbeschwerden lindern können. Für diesen Zweck werden auch Pflaster vorgeschlagen und verkauft, die ätherische Öle enthalten und diese über einen bestimmten Zeitraum freigeben. Solche Pflaster können jedoch nicht in direktem Hautkontakt angewandt und in der Regel insbesondere auch nicht auf die Haut unterhalb der Nase geklebt werden. Dies rührt daher, dass ätherische Öle die nachteilige Eigenschaft einer Hautreizung aufweisen, und dass bei Schnupfenbeschwerden die Haut unterhalb und um die Nase durch den Gebrauch von Taschentüchern ohnehin bereits gereizt und gerötet ist. Daher werden im Allgemeinen Pflaster, die ätherische Öle enthalten, nicht unter die Nase, sondern z.B. auf die Nachtbekleidung oder auch auf die Brust geklebt. Dies hat wiederum den Nachteil, dass das Reservoir mit den ätherischen Ölen zu weit von der Nase entfernt ist, und die Öle ihre Wirkung somit nicht optimal entfalten können.

Die DE 698 21 107 T2 (EP 0996407 B1) beschreibt Klebepflaster zur direkten Aufbringung auf die menschliche Haut. Dieses besteht aus einem Substrat, hergestellt aus Gewebe oder aus einer dünnen Folie aus synthetischem Material wie Polyesterkunststoff oder Weich-PVC, welches zum Aufkleben auf die Haut mit einer Klebstoffschicht aus z.B. Acrylklebstoff versehen ist. Die andere Oberfläche des Substrats kann ebenfalls einen Klebstofffilm tragen und ist mit Mikrokapseln beschichtet, welche ätherische Öle enthalten. Die Mikrokapseln sind mit Hilfe eines wasserlöslichen Harzes, bestehend aus Acryl- oder Polyvinylharzen, auf der Substratoberfläche verankert. Diese Pflaster können z.B. unter den Achselhöhlen angebracht werden, wo die Mikrokapseln durch die Armbewegung brechen und ihren Inhalt freisetzen. Auch können diese Pflaster als Nasenpflaster verwendet und an bzw. unter der Nase angebracht werden. Da das verwendete Substrat die angegriffene und entzündete Haut nicht vor einem Kontakt mit den in den Mikrokapseln enthaltenden ätherischen Ölen nach dem Aufbrechen der Kapseln schützt, und das Brechen der Kapseln durch die Person, die das Pflaster trägt, vorsätzlich verursacht werden muss, liegen die Nachteile eines solchen therapeutischen Systems unmittelbar auf der Hand.

Aufgabe der vorliegenden Erfindung war daher die Bereitstellung eines therapeutischen Systems, bevorzugt in Form eines (therapeutischen) Pflasters oder pflasterförmigen Arzneimittels, welches die Nachteile des Standes der Technik nicht oder nur in weit geringerem Ausmaß aufweist.

Diese Aufgabe wird durch das nachfolgend und in den Ansprüchen beschriebene Pflaster in überraschender Weise gelöst.

Dieses Pflaster dient zur Fixierung im direkten Hautkontakt unter der Nase, wo es über mehrere Stunden die enthaltenen ätherischen Öle freisetzen kann, wodurch die Nasenlöcher erweitert gehalten werden.

Gegenstand der vorliegenden Erfindung ist ein Pflaster (Heftpflaster zur direkten Anbringung auf die menschliche Haut), bevorzugt in Form eines so genannten Nasenpflasters und insbesondere zur Behandlung von Schnupfenbeschwerden, welches, ausgehend von der Hautoberfläche, zumindest folgende Schichten aufweist oder bevorzugt aus diesen Schichten besteht:
(a) mindestens eine haftklebende und hautfreundliche Polymerschicht,
(b) mindestens eine für ätherische Öle undurchlässige Zwischenschicht,
(c) mindestens eine weitere haftklebende Polymerschicht (auf der der Haut abgewandten Oberfläche der Zwischenschicht) und
(d) mindestens eine ätherische Öle enthaltende Rückschicht (als äußerste Schicht).

Bevorzugt weist das erfindungsgemäße Pflaster die genannten Schichten jeweils einmal auf, und es besteht bevorzugt aus den genannten Schichten. Die beiden genannten haftklebenden Schichten können bzgl. ihrer Zusammensetzung und/oder Dicke gleich oder verschieden sein. Die auf der Haut aufzubringende haftklebende Polymerschicht ist bevorzugt zusätzlich mit einer Schutzschicht aus z.B. silikonisiertem Papier oder silikonisierter Folie (z.B. PET-Folie) versehen. Die einzelnen Pflaster sind in üblicher Weise verpackt, z.B. in einem Vierrandsiegelbeutel aus Verbundpackstoff aus Polyacrylnitril , Aluminiumfolie und Papier.

Die im Hautkontakt stehende haftklebende und hautfreundliche Polymerschicht (a) enthält bevorzugt Polyacrylate, Polyisobutylene und/oder Polysiloxane in der Form der Homo- oder Copolymere. Bevorzugt besteht diese Schicht aus einer oder mehreren der genannten Substanzen. Besonders bevorzugt enthält oder besteht diese Polymerschicht aus einem oder mehreren Vinylacetat-Acrylatcopolymeren, insbesondere aus solchen mit einer Säurezahl von kleiner als 1.

Es ist ein besonderes Merkmal des erfindungsgemäßen Pflasters, dass die im Hautkontakt stehende, haftklebende und hautfreundliche Polymerschicht (a) Dexpanthenol enthalten kann und in einer bevorzugten Ausführungsform auch enthält.

Die für ätherische Öle undurchlässige Zwischenschicht (b) kann aus verschiedenen Polymeren bestehen. Bevorzugt sind hier Polyester und von diesen insbesondere Polyethylenterephthalat, oder - wenn auch weniger bevorzugt - Polyolefine wie Polypropylen.

Die die ätherischen Öle enthaltende Rückschicht besteht bevorzugt aus Vliesen. Insgesamt kann das Pflaster in jedweder Form zur Verfügung gestellt werden, wobei für pädiatrische Anwendungen Tiersilhouetten bevorzugte Ausführungsformen darstellen.

Haftklebende Polymere sind im Stand der Technik zwar bekannt, sie gewährleisten in der Regel aber die Fixierung von Pflastern oder pflasterförmigen Arzneimitteln wie beispielsweise transdermalen therapeutischen Systemen (TTS) nur dann, wenn sie klebrig machende Harze, wie zum Beispiel Kolophonium oder seine Derivate, enthalten. Diese Harze sind Naturprodukte und wechseln daher in ihrer quantitativen und qualitativen Zusammensetzung. Dies ist für die Anwendung auf intakter Haut zwar noch akzeptabel, für eine Applikation auf gereizter Haut, wie diese insbesondere bei Schnupfen- und Erkältungsbeschwerden auftritt, aber nicht.

Gut haftende Polymere sind beispielsweise Polyacrylate. Besonders gut kleiben auf der Haut solche Polyacrylate, die durch Verwendung von freier Acrylsäure hergestellt wurden. Besonders geeignet sind sie dann, wenn ihre Säurezahl zwischen 0,5 und 7,5 liegt. Bei niedrigeren Säurezahlen ist die Haftung, die durch die Wechselwirkung der sauren Gruppen des Polymers mit den sauren Bestandteilen der Haut zustande kommt, zu niedrig. Höhere Säurezahlen führen zu Hautreizungen. Bevorzugt sind Polymere mit Säurezahlen von kleiner als 1.

Die gleichen Ausführungen gelten grundsätzlich auch für Polyisobutylene und Polysilioxane, wobei bei diesen Polymeren saure Monomere nicht verwendet werden können. Sie haften daher nur dann dauerhaft, wenn sie mit Klebrigmachern, den erwähnten Kolophoniumderivaten, kombiniert werden.

Dexpanthenol (auch als Pantothenol, D-Panthenol oder Panthenol bezeichnet) wird in der Kosmetik und in Arzneimitteln zur beschleunigten Wundheilung eingesetzt. Dieser Wirkstoff ist Inhaltsstoff für Hautcremes, Salben, Lutschtabletten, Nasensprays, Augentropfen und Kontaktlinsenreinigungsprodukte. Eine Verwendung als Klebrigmacher, insbesondere im Zusammenhang mit Heftpflastern, ist im Stand der Technik nicht beschrieben. Es zeigte sich überraschenderweise, dass Dexpanthenol die Klebkraft von Polymeren, die zur Herstellung von TTS, Wundschnellverbänden, Heftpflastern allgemein und auch für die erfindungsgemäßen Pflaster (Polymerschicht (a)) verwendet werden, deutlich erhöht. Dies gilt insbesondere für sogenannte neutrale Polymere, wie z.B. Polyisobutylene und Polysiloxane. Aber auch bei den erfindungsgemäßen Polyacrylat Homo- und Copolymeren lassen sich durch den Einsatz von Dexpanthenol entsprechende Verbesserungen erzielen. Besonders erstaunlich ist hierbei, dass Haftkleber bzw. haftklebende Polymerschichten (z.B. Schicht (a)), welche Dexpanthenol enthalten, rückstandsfrei und ausgesprochen sanft von der - gereizten und überempfindlichen - Haut entfernt werden können, was besonders in der Pädiatrie einen entscheidenden Vorteil darstellt. Darüber hinaus entfaltet das Dexpanthenol neben seiner Wirkung als Klebrigmacher natürlich auch seine wundheilende Wirkung, was insbesondere der gereizten und empfindlichen Haut bei der Behandlung von Schnupfenbeschwerden zusätzlich zugute kommt.

Ätherische Öle sind lipophile flüssige Pflanzenbestandteile mit angenehmem Eigengeruch. Wenn Substanzen riechen, haben sie einen hohen Dampfdruck, was bedeutet, dass sie ein Molekulargewicht <500 Dalton haben müssen. Für solche Verbindungen stellt menschliche Haut keine Barriere dar, was besonders für nicht intakte Haut gilt. Daher sollen die ätherischen Öle nicht auf die durch Verwendung von Taschentüchern gereizte Haut gelangen, sollen aber gleichzeitig nahe den Nasenlöchern appliziert werden. Die sanft klebende hautfreundliche Polymerschicht ist daher frei von ätherischen Ölen und auf der der Haut abgewandten Oberfläche mit einer Zwischenschicht (b) abgedeckt, die eine Barriere für ätherische Öle darstellt. Hierzu kommen dünne Metallfolien aus beispielsweise Aluminium, aber auch Polymerfolien, zum Beispiel Polyester wie Polyethylenterephtalat, in Frage.

Bevorzugte ätherische Öle sind solche, die sich zur Behandlung von Schnupfen- und Erkältungsbeschwerden eignen, sie sind dem Fachmann hinreichend bekannt und in der Literatur beschrieben (z.B. U. Bühring, "Praxis-Lehrbuch der modernen Heilpflanzenkunde: Grundlagen - Anwendung -Therapie", Georg Thieme Verlag). Als Beispiele ohne Anspruch auf Vollständigkeit seien genannt: Eukalyptol, Kiefer, Menthol, Minze, Orangenblüte, Lavendel, Citronella, Paciulli, Salbei, Ylang-Ylang usw. Mischungen mit ausgezeichneter aromatischer Wirkung können z.B. aus 2 Gewichtsteilen Eukalyptol und 1 Gewichtsteil Pinie oder auch aus 2 Teilen Menthol und 1 Teil Minze hergestellt werden.

Die andere Seite der für ätherische Öle undurchlässigen Zwischenschicht (b) wird mit einem Haftkleber (c) beschichtet, der die Rückschicht (d) mit den ätherischen Ölen fixieren soll. Dies kann der gleiche Kleber sein, der den Hautkontakt sicherstellt (Schicht (a)), es kann aber auch ein anderer (kostengünstigerer) sein, weil er keinen direkten Hautkontakt hat. Beispielsweise können Polyisobutylene, Polyacrylate, und EVA-Copolymere verwendet werden.

Die die ätherischen Öle enthaltende Rückschicht kann z.B. aus Papier oder saugfähigem Vlies, zum Beispiel aus TWE Vlies, bestehen. Materialien, die in Frage kommen sind dem Fachmann bekannt. Das erfindungsgemäß bevorzugte TWE Vlies gibt es in verschiedenen Ausführungen und ist im Handel erhältlich (TWE Dierdorf, BRD). Z.B. die Para Therm^{®} Produktlinie, bei der es sich um ein durch Druck und Hitze (Heißluft) verfestigtes Vlies aus thermoplastischen Fasern wie Polypropylen/Viscose handelt.

Das erfindungsgemäße Pflaster bzw. Nasenpflaster kann in der Pädiatrie oder auch für Erwachsene eingesetzt werden. Sollen Kinder therapiert werden, so sollten die ätherischen Öle weder Campher noch Menthol enthalten. Für Erwachsene gelten diese Vorbehalte nicht.

Sollen Kinder therapiert werden, so ist es grundsätzlich möglich, das Pflaster in Tierform, zum Beispiel als Krokodil oder Dinosaurier, auszustanzen. Andere Formen sind möglich und fallen daher auch unter die vorliegende Erfindung.

Nachfolgend werden einige bevorzugte Merkmale für das erfindungsgemäße Pflaster näher beschrieben:
- Der Gewichtsanteil von Dexpanthenol in der haftklebenden Schicht (a) liegt zweckmäßig bei 0,5 und 25 Gew.-%, vorzugsweise bei 2 bis 15 Gew.-%.
- Die Schichtdicken der Schichten (a) - (d) liegen zweckmäßigerweise jeweils bei 30 bis 280 µm, vorzugsweise 50 bis 150 µm.

Die nachfolgenden Beispiele dienen zur näheren Erläuterung der Erfindung, ohne diese sachlich zu beschränken. Vielmehr ist jede für den Fachmann sinnvolle Kombination von (einzelnen) Merkmalen, unabhängig davon, ob sie in der Beschreibung, den Beispielen oder Ansprüchen genannt sind, von der vorliegenden Erfindung umfasst.

Allgemeine Vorgehensweise:
1. Kleber für den Hautstrich:
   Eine im Handel erhältliche Lösung von Vinylacetat-Acrylat-copolymerisat in Etylacetat/Heptan wird großtechnisch hergestellt, in dem die Monomere Vinylacetat, Glycidylmethacrylat, 2-Hydroxyethylacrylat und 2-Ethylhexylacrylat im Gewichtsverhältnis 5:0,15:5:89,85 gemischt, in Ethylacetat gelöst und mit Azoisobutyrodinitril (AIBN) zur Polymerisation gebracht werden. Nach beendeter Polymerisation wird mit Heptan so verdünnt, dass eine ca. 50 gewichtsprozentige Lösung des Polymers im Lösungsmittelgemisch Ethylacetat/Heptan entsteht. Vor der Verwendung in der Beschichtung zur Herstellung der Pflaster wird so viel einer Lösung von mittelkettigen Triglyceriden (DAB) in Ethylacetat und einer Lösung von Dexpanthenol (Ph.Eur.) in Ethanol zur im Handel erhältlichen Lösung von Vinylacetat-Acrylat-copolymerisat in Ethylacetat/Heptan zugegeben, dass Triglyceride, Dexpanthenol und Polymer im Gewichtsverhältnis 2:5:93 vorliegen.
   Man rührt bei Raumtemperatur bis zur Homogenität.
   Diese Polymerlösung wird für die Herstellung der der Haut zugewandten Seite (Schicht (a)), im Weiteren der Hautstrich genannt, verwendet.
2. Kleberlösung für die Zwischenschicht
   Kleberlösung ist die oben erwähnte im Handel erhältliche Lösung von Vinyl-acetat-Acrylat-copolymerisat in Ethylacetat/Heptan.
   Pflasterherstellung
   200 g des Klebers für den Hautstrich werden so auf eine silikonisierte PET-Folie (Schutzfolie) beschichtet, dass nach Abdampfen der Lösemittel im Umlufttrockenschrank bei maximal 80 °C eine Kleberschicht mit einem Flächengewicht von ca. 50 g/m² entsteht.
   Die haftklebende Oberfläche wird mit einer sehr dünnen PET Folie in der Dicke von 6 µm abgedeckt (Zwischenschicht (b)).
   Die andere Seite der 6 µm PET Folie wird mit der Kleberlösung für die Zwischenschicht abgedeckt. Wie beschrieben werden durch Trocknen im Trockenschrank die Lösungsmittel entfernt. Man erhält einen Kleberauftrag von ca. 20g/m² und deckt die klebende Oberfläche (c) mit einem TWE Vlies ab.
   Man stanzt nun längliche Gebilde mit einem geeigneten Werkzeug so aus, dass alle Schichten außer der 100 µm silikonisierten PET-Schutzfolie durchtrennt werden. Das zwischen den einzelnen Pflastern liegende Gitterwerk aus Hautstrich, PET Folie 6 µm, Kleberstrich für die Zwischenschicht und TWE Vlies wird abgezogen und verworfen.
   Die PET Schutzfolie überragt demzufolge die anderen Schichten und wird in Rechtecke geschnitten. Anschließend tropft man ca. 50 µl von ätherischem Öl auf das TWE Vlies und siegelt die einzelnen Pflaster in Vierrandsiegelbeutel aus Verbundpackstoff aus Polyacrylnitril, Aluminiumfolie und Papier.
   Wie erwähnt werden für Kinder ätherische Öle ohne Campher oder Menthol, zum Beispiel Fichtennadelöl, Kiefernadelöl, Kamille oder andere verwendet.

### Beispiel 1

630 kg der Polymerlösung (a) werden auf einer industriellen Beschichtungsanlage auf silikonisiertes Papier beschichtet und in eine Trockenanlage gefahren. Da Papier verwendet wird, darf aus naheliegenden, dem Fachmann bekannten Gründen, die Trockentemperatur 80 °C nicht übersteigen. Das Flächengewicht beträgt etwa 100g/m². Nach beendeter Trocknung wird das Laminat aus Papier und haftklebender Polymerschicht mit PET Folie abgedeckt.

Nach Entfernen des Papiers wird die Wasserdampfdurchlässigkeit mit ca. 150g/m² x 24 h bestimmt.

Routinemäßig durchgeführte gaschromatographische Untersuchungen auf niedermolekulare Verunreinigungen aus dem Herstellungsprozess ergeben folgende Ergebnisse, die in den Tabellen 1 bis 3 zusammengefasst sind (DL= "detection limit"):

**Tab. 1 Lösemittel**

| Lösemittel | analytisches Ergebnis | Spezifikation gemäß Ph.Eur. |
|---|---|---|
| Ethanol | <DL | max 0,5 % |
| Heptan | <DL | max 0,5 % |
| Ethylacetat | <DL | max 0,5 % |

**Tab. 2 Monomere**

| Monomer | analytisches Ergebnis |
|---|---|
| Acrylsäure | <DL |
| Butylacrylat | <DL |
| Vinylacetat | <DL |
| 2-Ethylhexylacrylat | <0,15 % |

**Tab. 3 Reste vom Radikalstarter und Vernetzer**

| TMSN | ca. 300 ppm |
|---|---|
| AcetylAcetonat | <DL |

Aufgrund dieser Analysenergebnisse spricht nichts gegen eine Verwendung des Materials für Pflaster, um Wundschnellverbände oder TTS auf menschlicher Haut zu fixieren.

Konzentrationen an TMSN und 2-Ethylhexylacrylat scheinen für eine Verwendung auf nicht intakter Haut aber noch zu hoch.

Daher werden die Produktionsbedingungen für Beispiel 2 geändert.

### Beispiel 2

630 kg der Polymerlösung (a) werden auf einer industriellen Beschichtungsanlage auf silikonisierte PET Folie beschichtet und in eine Trockenanlage gefahren. Da PET verwendet wird, darf aus naheliegenden, dem Fachmann bekannten Gründen, die Trockentemperatur sogar bis auf 115 °C ansteigen. Das Flächengewicht beträgt etwa 100 g/m². Nach beendeter Trocknung wird das Laminat aus PET und haftklebender Polymerschicht mit PET Folie (b) abgedeckt.

Gaschromatographische Untersuchungen auf niedermolekulare Verunreinigungen aus dem Herstellungsprozess ergeben folgende Ergebnisse, die in den Tabellen 4 bis 6 zusammengefasst sind:

**Tab. 4 Lösemittel**

| Lösemittel | analytisches Ergebnis | Spezifikation gemäß Ph.Eur. |
|---|---|---|
| Ethanol | <DL | max 0,5 % |
| Heptan | <DL | max 0,5% |
| Ethylacetat | <DL | max 0,5 % |

**Tab. 5 Monomere**

| Monomer | analytisches Ergebnis |
|---|---|
| Acrylsäure | <DL |
| Butylacrylat | <DL |
| Vinylacetat | <DL |
| 2-Ethylhexylacrylat | <300 ppm |

**Tab. 6 Reste vom Radikalstarter und Vernetzer**

| TMSN | <100 ppm |
|---|---|
| AcetylAcetonat | <DL |

Aufgrund dieser Analysenergebnisse werden Tests zur Geno- und Cytotoxizität durchgeführt, um eine Verwendung des Materials für Pflaster für eine Fixierung von Halbimplantaten auf nicht intakter Haut zu rechtfertigen.

Das Pflaster gemäß Beispiel zeigt sich als weder geno- noch cytotoxisch.

Die Klebkraft von Stanzlingen mit der Fläche 72 mm x 72 mm wird mit einer Methode in Anlehnung an die Methoden der AFERA Norm an einer Stahlplatte und einem Abzugswinkel von 90° mit ca. 58 N/Stanzling bestimmt.

Aufgrund der Analysenergebnisse zur Geno- und Cytotoxität, der Wasserdampfdurchlässigkeit und der außerordentlich hohen Klebkraft ist insbesondere das Pflaster gemäß Beispiel 2 zur Fixierung von Halbimplantaten auf nicht intakter Haut geeignet.

## Patentansprüche

1. Pflaster, welches zumindest folgende Schichten aufweist
(a) mindestens eine haftklebende und hautfreundliche Polymerschicht,
(b) mindestens eine für ätherische Öle undurchlässige Zwischenschicht,
(c) mindestens eine weitere haftklebende Polymerschicht und
(d) mindestens eine ätherische Öle enthaltende Rückschicht.

2. Pflaster nach Anspruch 1, **dadurch gekennzeichnet, dass** es die Schichten (a) bis (d) jeweils einmal aufweist.

3. Pflaster nach Anspruch 2, **dadurch gekennzeichnet, dass** es die Schichten (a) bis (d) jeweils einmal aufweist und aus diesen besteht.

4. Pflaster nach einem der Ansprüche 1 bis 3, **dadurch gekennzeichnet, dass** die Schicht (a) Dexpanthenol als Klebrigmacher enthält.

5. Pflaster nach Anspruch 4, **dadurch gekennzeichnet, dass** der Anteil von Dexpanthenol in der Schicht (a) 0,5 bis 25,0 Gew. -%, bezogen auf das Gewicht der Schicht (a), beträgt.

6. Pflaster nach einem der Ansprüche 1 bis 5, **dadurch gekennzeichnet, dass** die Polymerschicht (a) Polyacrylate, Polyisobutylen und /oder Polysiloxane in Form von Homo- und/oder Copolymeren enthält oder aus diesen besteht.

7. Pflaster nach einem der Ansprüche 1 bis 6, **dadurch gekennzeichnet, dass** die Polymerschicht (a) ein Vinyl-Acrylatcopolymer enthält oder aus diesem besteht.

8. Pflaster nach Anspruch 6 oder 7, **dadurch gekennzeichnet, dass** die Polyacrylate oder das Vinyl-Acrylatcopolymer eine Säurezahl von kleiner 1 aufweisen.

9. Pflaster nach einem der Ansprüche 1 bis 8, **dadurch gekennzeichnet, dass** die Zwischenschicht (b) aus Polyethylenterephthalat besteht.

10. Pflaster nach einem der Ansprüche 1 bis 9, **dadurch gekennzeichnet, dass** die Rückschicht (d) aus einem oder mehreren Vliesen besteht oder ein oder mehrere Vliese aufweist.

11. Pflaster nach Anspruch 10, **dadurch gekennzeichnet, dass** die Rückschicht (d) aus einem Vlies besteht.

12. Verwendung eines Pflasters nach einem der Ansprüche 1 bis 11 als Nasenpflaster zur Behandlung von Schnupfenbeschwerden.

13. Verwendung von Dexpanthenol als Klebrigmacher in haftklebenden Polymerschichten.

14. Verwendung nach Anspruch 13 in einem Pflaster nach einem der Ansprüche 1 bis 11.
